# EUROPEAN PATENT APPLICATION

(11) **EP 1 743 902 A1**
(43) Date of publication of application: **17.01.2007**
(21) Application number: 05728917.5
(22) Date of filing: 31.03.2005
(51) Int. Cl.: C07J 5/00

(54) **METHOD FOR PRODUCING 5ALPHA-PREGNANE DERIVATIVE**

(30) Priority: 31.03.2004 JP 2004108419
(71) Applicant: Kuraray Co., Ltd., Okayama 710-8622 (JP)
(72) Inventor: SUGIOKA, Takashi, Kurashiki-shi, Okayama 7100801 (JP); OHZONO, Shigeo, Kurashiki-shi, Okayama 7100801 (JP); KOYAKUMARU, Kenichi, Bizen-shi, Okayama 7050025 (JP); NAKAGAWA, Naoshi, Tokyo 1008115 (JP)
(74) Representative: Duckworth, Timothy John
(86) International application number: PCT/JP2005/006828
(87) International publication number: WO 2005/095434

(57) **Abstract**

The present invention relates to a method of producing 5α,-pregnane derivatives represented by the formula (II), which is **characterized by** reacting a pregnane derivative represented by the formula (I) with a metal selected from alkali metals and alkaline earth metals in the presence of a proton donor and an amine and/or ammonia. According to the present invention, a method capable of producing 5α-pregnane derivatives useful as synthetic intermediates for squalamine, in a high yield from easily available raw materials, can be provided: 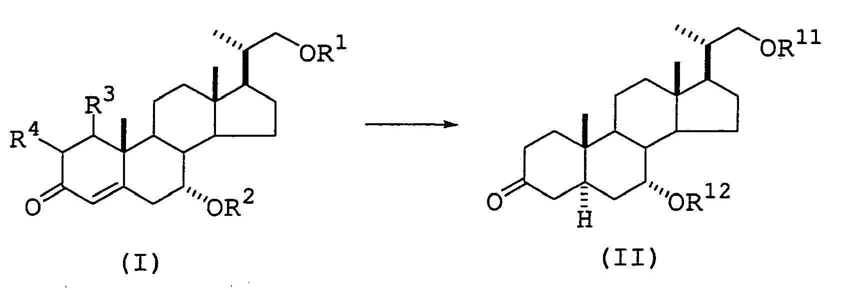 wherein R¹ is a hydroxyl-protecting group, and R², R¹¹ and R¹² are each independently a hydrogen atom or a hydroxyl-protecting group and R³ and R⁴ are each hydrogen atoms in combination form a bond.

## Description

### Technical Field

The present invention relates to production methods of 5_{α}-pregnane derivatives useful as synthetic intermediates for squalamine.

### Background Art

Squalamine is a compound represented by the formula (V): 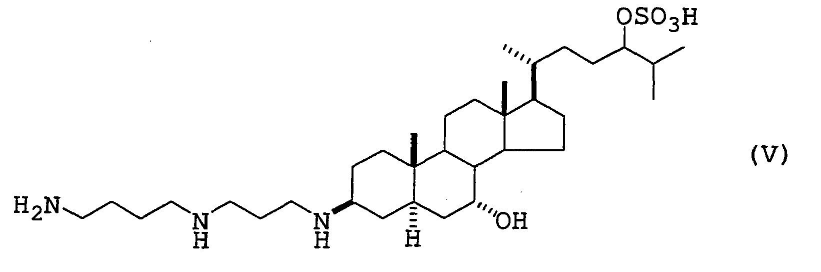 , which has been reported to show strong antibacterial activity against Gram-positive bacteria, Gram-negative bacteria, fungi and the like, as well as anticancer activity, and is drawing attention as a new antibiotic.

Conventionally, squalamine is extracted from the liver of dogfish. In view of its extremely low content of 0.001-0.002 wt% and poor extraction efficiency, however, various chemical synthetic methods have been studied. Particularly, (20S)-7α,21-dihydroxy-20-methyl-5α,-pregn-3-one represented by the formula (IV): 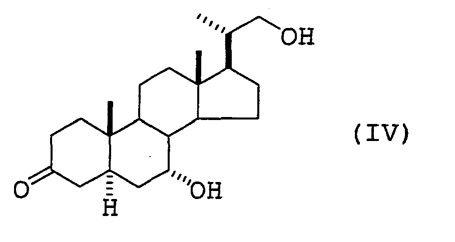 (WO01/79255, and Organic Letters, Vol. 2, p. 2921 (2000)) and (20S)-21-tert-butyldimethylsilyloxy-7α-hydroxy-20-methyl-5α-pregn-3-one represented by the formula (VI): 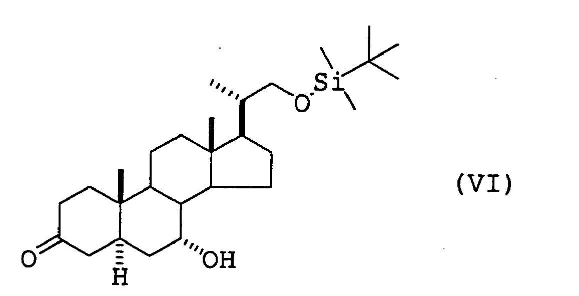 (WO03/51904) are known to be useful synthetic intermediates that can be converted to squalamine comparatively in a few steps.

Conventionally, as production methods of (20S)-7α,21-dihydroxy-20-methyl-5α-pregn-3-one, a method (WO01/79255) including subjecting (20S)-7α,21-dihydroxy-20-methylpregn-4-en-3-one to what is called the Birch reduction using not less than 30 equivalents of metal lithium in liquid ammonia with the aim of stereoselective reduction to an α form in the 5-position, a method (WO02/20552) including subjecting (20S)-7α,21-dihydroxy-20-methylpregna-1,4-dien-3-one to the Birch reduction using 10 equivalents of metal lithium in liquid ammonia, and the like have been developed.

In addition, as a production method of (20S)-21-tert-butyldimethylsilyloxy-7α,-hydroxy-20-methyl-5α-pregn-3-one, a method (WO03/51904) including reducing (20S)-7α,21-dihydroxy-20-methylpregna-1,4-dien-3-one in the same manner as in the above to give (20S)-7α,21-dihydroxy-20-methyl-5α-pregn-3-one, and then protecting the hydroxyl group at the 21-position of the compound with a tert-butyldimethylsilyl group is known.

### Disclosure of the Invention

However, the yield of the above-mentioned method is 71% at the highest and, in consideration of the fact that the pregnane derivative is an expensive raw material, the method cannot be considered a preferable production method but has a room for improvement before its industrial practice.

Therefore, an object of the present invention is to provide a method of efficiently producing (20S)-7α,21-dihydroxy-20-methyl-5α,-pregn-3-one useful as an synthetic intermediate for squalamine and a (20S)-7α,21-dihydroxy-20-methyl-5α-pregn-3-one derivative wherein the hydroxyl group(s) at the 21-position and/or the 7-position are/is protected with protecting group(s), which comprises stereoselectively reducing a (20S)-7α,21-dihydroxy-20-methylpregna-1,4-dien-3-one derivative or a (20S)-7α,21-dihydroxy-20-methylpregn-4-en-3-one derivative to a 5α, form, and then, where necessary, eliminating the hydroxyl-protecting group(s) of the 5α form.

In the aforementioned conventional reaction, a ketone derivative stereoselectively converted to a 5α form is obtained from an unsaturated ketone having at least the carbon-carbon double bond at 4- and 5-positions as a raw compound, which corresponds to what is called the partial reduction where an unsaturated ketone is converted to a saturated ketone. According to the conventional reaction, however, it has been clarified that the saturated ketone is further reduced to give an alcohol form due to the side reaction. To suppress such side reaction, it is important that the reaction be carried out using a reducing agent in an amount close to the equivalent amount, which is necessary for the partial reduction. However, in the conventional reaction, a large excess of metal lithium is used.

The present inventors have conducted intensive studies of the reason for low yields by conventional methods and found that since a hydroxyl group is present at the 21-position of the raw material pregnane derivative, namely, since metal lithium, which is a reducing agent, is decomposed due to the highly reactive primary hydroxyl group present at the 21-position to lose reducing ability, use of excess metal lithium is unavoidable, and that since the primary hydroxyl group also acts as a proton donor during the reduction reaction, which in turn further promotes the reaction, an alcohol form is by-produced.

Based on such finding, a reaction was carried out using a compound wherein the hydroxyl group at the 21-position is protected as a raw compound. As a result, the decomposition of the reducing agent due to the hydroxyl group and the action as a proton donor were suppressed. As a result, the amount of the reducing agent to be used can be decreased, the side reaction can be suppressed and the yield of the objective 5α-pregnane derivative can be increased, thus solving the problems of the conventional methods.

Accordingly, the present invention provides the following.
(1) A method for producing a 5_{α}-pregnane derivative represented by the formula (II): 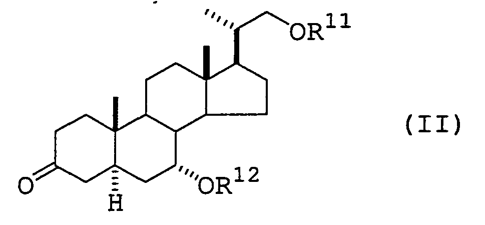 wherein R¹¹ and R¹² are each independently a hydrogen atom or a hydroxyl-protecting group (hereinafter sometimes to be referred to as compound (II) in the present specification), which comprises reacting a pregnane derivative represented by the formula (I): 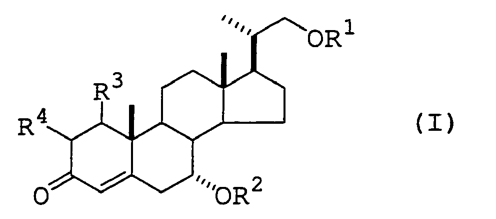 wherein R¹ is a hydroxyl-protecting group, R² is a hydrogen atom or a hydroxyl-protecting group, and R³ and R⁴ are each a hydrogen atom or in combination form a bond (hereinafter sometimes to be referred to as compound (I) in the present specification), with a metal selected from alkali metals and alkaline earth metals in the presence of a proton donor and an amine and/or ammonia.
(2) The method of the above-mentioned (1), wherein R² and R¹² are hydrogen atoms.
(3) The method of the above-mentioned (1) or (2), wherein R³ and R⁴ in combination form a bond.
(4) The method of the above-mentioned (3), wherein R¹ and R¹¹ are tri-substituted silyl groups having three, same or different, substituents selected from the group consisting of an alkyl group optionally having substituent(s), an aryl group optionally having substituent(s), an alkoxyl group optionally having substituent(s) and an aryloxy group optionally having substituent (s).
(5) The method of the above-mentioned (4), wherein R¹ and R¹¹ are tert-butyldimethylsilyl groups.
(6) The method of any one of the above-mentioned (1)-(5), wherein the metal is an alkali metal.
(7) The method of the above-mentioned (6), wherein the alkali metal is lithium.
(8) A method for producing (20S)-7α,21-dihydroxy-20-methyl-5α-pregn-3-one represented by the formula (IV): 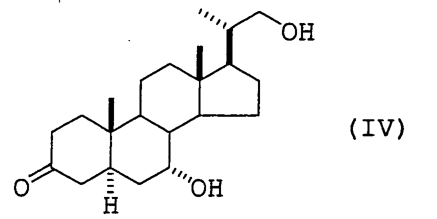 (hereinafter sometimes to be referred to as compound (IV) in the present specification), which comprises the steps of
   (a) reacting compound (I) with a metal selected from alkali metals and alkaline earth metals in the presence of a proton donor and an amine and/or ammonia to give a 5α-pregnane derivative represented by the formula (III): 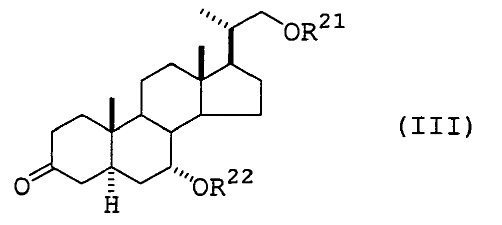 wherein R²¹ is a hydroxyl-protecting group and R²² is a hydrogen atom or a hydroxyl-protecting group (hereinafter sometimes to be referred to as compound (III) in the present specification); and
   (b) eliminating the hydroxyl-protecting group of compound (III) obtained by the aforementioned step.
(9) The method of the above-mentioned (8), wherein R² and R²² are hydrogen atoms.
(10) The method of the above-mentioned (8) or (9), wherein R³ and R⁴ in combination form a bond.
(11) The method of the above-mentioned (10), wherein R¹ and R²¹ are tri-substituted silyl groups defined above.
(12) The method of the above-mentioned (11), wherein R¹ and R²¹ are tert-butyldimethylsilyl groups.

According to the method of the present invention, by using a compound wherein the hydroxyl group at the 21-position is protected as a raw compound for producing a 5α-pregnane derivative by stereoselectively reducing a pregn-4-ene derivative or pregna-1,4-diene derivative, a 5α-pregnane derivative useful as a synthetic intermediate for squalamine can be produced in a high yield. According to the method of the present invention, moreover, excessive use of a reducing agent as in the conventional methods becomes unnecessary, which in turn obliterates side reactions and provides a beneficial economical effect.

### Best Mode for Embodying the Invention

### 1. Explanation of symbols

In the above-mentioned formulas, the hydroxyl-protecting group represented by R¹, R², R¹¹, R¹², R²¹ or R²² may be any as long as it acts as a hydroxyl-protecting group and, for example, an alkyl group optionally having substituent(s); an acyl group optionally having substituent(s) (e.g., a formyl group, an alkylcarbonyl group optionally having substituent(s), an alkenylcarbonyl group optionally having substituent(s), an arylcarbonyl group optionally having substituent(s) etc.); an alkoxycarbonyl group optionally having substituent(s); an aryloxycarbonyl group optionally having substituent(s); a carbamoyl group (e.g., a carbamoyl group wherein the nitrogen atom is optionally substituted by an alkyl group optionally having substituent(s) or an aryl group optionally having substituent(s)); a tri-substituted silyl group (the tri-substituted silyl group has three, same or different, substituents selected from the group consisting of an alkyl group optionally having substituent(s), an aryl group optionally having substituent(s), an alkoxyl group optionally having substituent(s) and an aryloxy group optionally having substituent(s)); and the like can be mentioned.

The alkyl group as the hydroxyl-protecting group represented by R¹, R², R¹¹, R¹², R²¹ or R²²; the alkyl group as a part of the acyl group and the alkyl group as a substituent that the acyl group optionally has; the alkyl group as a part of the alkoxycarbonyl group; the alkyl group as a substituent that the carbamoyl group optionally has; the alkyl group that the tri-substituted silyl group has, the alkyl group as a part of the alkoxyl group that the tri-substituted silyl group has, and the alkyl group as a substituent that the aryl group and aryloxy group that the tri-substituted silyl group has optionally have, may be linear, branched or cyclic, and preferably has 1 to 12, more preferably 1 to 8, carbon atoms. As such alkyl group, for example, methyl group, ethyl group, propyl group, isopropyl group, butyl group, isobutyl group, tert-butyl group, hexyl group, octyl group, dodecyl group, cyclopentyl group, cyclohexyl group and the like can be mentioned.

The above-mentioned alkyl group optionally has substituent(s). While the number of substituents is not particularly limited, 1 to 6 is preferable, and when the number is two or more, the substituents may be the same or different. As such substituent, for example, an aryl group having 6 to 12, preferably 6 to 10, carbon atoms, which optionally has substituent(s), such as phenyl group, tolyl group, methoxyphenyl group, nitrophenyl group, naphthyl group, fluorenyl group and the like; an alkenyl group having 2 to 12, preferably 2 to 10, carbon atoms such as vinyl group and the like, which optionally has substituent(s); a linear, branched or cyclic alkoxyl group having 1 to 12, preferably 1 to 8, carbon atoms (the alkoxyl group may form a ring structure (e.g., tetrahydropyran ring, tetrahydrofuran ring etc.) together with an alkyl group which is a hydroxyl-protecting group) such as methoxy group, ethoxy group, propoxy group, isopropoxy group, butoxy group, isobutoxy group, tert-butoxy group, hexyloxy group, octyloxy group, dodecyloxy group, cyclopentyloxy group, cyclohexyloxy group and the like; an aralkyloxy group having 7 to 12, preferably 7 to 11, carbon atoms such as benzyloxy group and the like; an alkenyloxy group having 2 to 12, preferably 2 to 8, carbon atoms such as allyloxy group and the like; an aryloxy group having 6 to 12, preferably 6 to 10, carbon atoms such as phenoxy group, nitrophenoxy group, naphthyloxy group and the like, which optionally has substituent(s); and the like can be mentioned.

The alkenyl group as a part of the acyl group as the hydroxyl-protecting group represented by R¹, R², R¹¹, R¹², R²¹ or R²², and the alkenyl group as a substituent that the acyl group optionally has; the alkenyl group as a substituent that the aryloxycarbonyl group optionally has; and the alkenyl group as a substituent that the aryl group, alkoxyl group and aryloxy group that the tri-substituted silyl group has optionally have, may be linear, branched or cyclic, and preferably has 2 to 12, more preferably 2 to 8, carbon atoms. As such alkenyl group, for example, vinyl group, 1-methylvinyl group, 1-propenyl group, 1-octenyl group, 1-dodecenyl group, 1-cyclopentenyl group, 1-cyclohexenyl group and the like can be mentioned.

The above-mentioned alkenyl group optionally has substituent (s). While the number of substituents is not particularly limited, 1 to 6 is preferable, and when the number is two or more, the substituents may be the same or different. As such substituent, for example, an aryl group having 6 to 12, preferably 6 to 10, carbon atoms, which optionally has substituent(s), such as phenyl group, tolyl group, methoxyphenyl group, nitrophenyl group, naphthyl group, fluorenyl group and the like; a linear, branched or cyclic alkoxyl group having 1 to 12, preferably 1 to 8, carbon atoms such as methoxy group, ethoxy group, propoxy group, isopropoxy group, butoxy group, isobutoxy group, tert-butoxy group, hexyloxy group, octyloxy group, dodecyloxy group, cyclopentyloxy group, cyclohexyloxy group and the like; an aralkyloxy group having 7 to 12, preferably 7 to 11, carbon atoms such as benzyloxy group and the like; an alkenyloxy group having 2 to 12, preferably 2 to 8, carbon atoms such as allyloxy group and the like; an aryloxy group having 6 to 12, preferably 6 to 10, carbon atoms such as phenoxy group, nitrophenoxy group, naphthyloxy group and the like, which optionally has substituent(s); and the like can be mentioned.

The aryl group as a part of the acyl group as the hydroxyl-protecting group represented by R¹, R², R¹¹ , R¹², R²¹ or R²², and the aryl group as a substituent that the acyl group optionally has; the aryl group as a part of the aryloxycarbonyl group and the aryl group as a substituent that the aryloxycarbonyl group optionally has; the aryl group as a substituent that the carbamoyl group optionally has; the aryl group that the tri-substituted silyl group has, the aryl group as a part of the aryloxyl group that the tri-substituted silyl group has, and the aryl group as a substituent that the aryl group, alkoxyl group and aryloxy group that the tri-substituted silyl group has optionally have, preferably have 6 to 10 carbon atoms and, for example, phenyl group, naphthyl group and the like can be mentioned.

The above-mentioned aryl group optionally has substituent(s). While the number of substituents is not particularly limited, 1 to 6 is preferable, and when the number is two or more, the substituents may be the same or different. As such substituent, for example, a linear, branched or cyclic alkyl group having 1 to 12, preferably 1 to 8, carbon atoms such as methyl group, ethyl group, propyl group, isopropyl group, butyl group, isobutyl group, tert-butyl group, hexyl group, octyl group, dodecyl group, cyclopentyl group, cyclohexyl group and the like; a linear, branched or cyclic alkoxyl group having 1 to 12, preferably 1 to 8, carbon atoms such as methoxy group, ethoxy group, propoxy group, isopropoxy group, butoxy group, isobutoxy group, tert-butoxy group, hexyloxy group, octyloxy group, dodecyloxy group, cyclopentyloxy group, cyclohexyloxy group and the like; a linear, branched or cyclic acyloxy group having 1 to 12, preferably 1 to 8, carbon atoms such as formyloxy group, acetyloxy group, propionyloxy group, butyryloxy group, isobutyryloxy group, valeryloxy group, isovaleryloxy group, pivaloyloxy group, hexanoyloxy group, octanoyloxy group, dodecanoyloxy group, cyclopentanecarbonyloxy group, cyclohexanecarbonyloxy group, benzoyloxy group, methoxybenzoyloxy group, nitrobenzoyloxy group and the like; nitro group; cyano group and the like can be mentioned.

Of the hydroxyl-protecting groups represented by R¹, R², R¹¹, R¹², R²¹ or R²², specific examples of the alkyl group optionally having substituent(s) include methyl group, ethyl group, tert-butyl group, methoxymethyl group, tert-butoxymethyl group, benzyloxymethyl group, 2-tetrahydropyranyl group, 2-tetrahydrofuranyl group, 1-ethoxyethyl group, 1-benzyloxyethyl group, benzyl group, p-methoxybenzyl group, p-nitrobenzyl group, trityl group and the like, with preference given to methyl group, ethyl group, methoxymethyl group, 2-tetrahydropyranyl group, 2-tetrahydropyranyl group and 1-ethoxyethyl group.

Of the hydroxyl-protecting groups represented by R¹, R², R¹¹, R¹², R²¹ or R²², specific examples of the acyl group include formyl group, acetyl group, propionyl group, butyryl group, isobutyryl group, valeryl group, isovaleryl group, pivaloyl group, hexanoyl group, octanoyl group, dodecanoyl group, cyclopentanecarbonyl group, cyclohexanecarbonyl group, methoxyacetyl group, crotonoyl group, cinnamoyl group, phenylacetyl group, phenoxyacetyl group, benzoyl group, methoxybenzoyl group, nitrobenzoyl group and the like, with preference given to formyl group, acetyl group, propionyl group and pivaloyl group.

Of the hydroxyl-protecting groups represented by R¹, R², R¹¹, R¹², R²¹ or R²², specific examples of the alkoxycarbonyl group optionally having substituent(s) include methoxycarbonyl group, ethoxycarbonyl group, propoxycarbonyl group, isopropoxycarbonyl group, butoxycarbonyl group, isobutoxycarbonyl group, tert-butoxycarbonyl group, hexyloxycarbonyl group, octyloxycarbonyl group, dodecyloxycarbonyl group, cyclopentyloxycarbonyl group, cyclohexyloxycarbonyl group, benzyloxycarbonyl group, p-methoxybenzyloxycarbonyl group, fluorenylmethoxycarbonyl group, p-nitrobenzyloxycarbonyl group, allyloxycarbonyl group and the like, with preference given to methoxycarbonyl group, ethoxycarbonyl group, isobutoxycarbonyl group and allyloxycarbonyl group.

Of the hydroxyl-protecting groups represented by R¹, R², R¹¹, R¹², R²¹ or R²², specific examples of the aryloxycarbonyl group optionally having substituent(s) include phenoxycarbonyl group, p-nitrophenoxycarbonyl group and the like, with preference given to phenoxycarbonyl group.

Of the hydroxyl-protecting groups represented by R¹, R², R¹¹, R¹², R²¹ or R²², specific examples of the carbamoyl group include carbamoyl group wherein any hydrogen atom that a nitrogen atom has is optionally substituted, for example, by a linear, branched or cyclic alkyl group having 1 to 12 carbon atoms such as methyl group, ethyl group, propyl group, isopropyl group, butyl group, isobutyl group, tert-butyl group, hexyl group, octyl group, dodecyl group, cyclopentyl group, cyclohexyl group and the like, an aralkyl group having 7 to 12 carbon atoms such as benzyl group and the like, an alkenyl group having 2 to 12 carbon atoms such as allyl group and the like or an aryl group having 6 to 10 carbon atoms, which optionally has substituent(s), such as phenyl group, methoxyphenyl group, naphthyl group and the like, and the like.

Of the hydroxyl-protecting groups represented by R¹, R², R¹¹, R¹², R²¹ or R²², specific examples of the tri-substituted silyl group include trimethylsilyl group, triethylsilyl group, triisopropylsilyl group, dimethylisopropylsilyl group, diethylisopropylsilyl group, tert-butyldimethylsilyl group, tert-butyldiphenylsilyl group, tribenzylsilyl group, tert-butylmethoxyphenylsilyl group and the like, with preference given to tert-butyldimethylsilyl group, triethylsilyl group and triisopropylsilyl group, and more preference given to tert-butyldimethylsilyl group.

As R¹, R¹¹ or R²¹, a tri-substituted silyl group is preferable, and a tert-butyldimethylsilyl group is more preferable.

Since the hydroxyl group at the 7-position in compound (I) is slow in reaction with a metal reducing agent due to steric hindrance and does not adversely influence the reaction, it may or may not be protected. However, since introductory reaction of the protecting group can be omitted, it is preferably not protected. Therefore, as R², R¹² and R²², hydrogen atoms are preferable.

In the formula (I), R³ and R⁴ preferably form a bond in combination. Here, forming a bond in combination means that the carbon atoms to which R³ and R⁴ are respectively bonded form a double bond.

### 2. Reduction method and reaction conditions (production method of compound (II) or compound (III) from compound (I))

The method for producing compound (II) or compound (III) from compound (I) of the present invention includes a step of reacting compound (I) with a metal such as alkali metals (e.g., lithium, sodium, potassium etc.), alkaline earth metals (e.g., magnesium, calcium, strontium, barium etc.) and the like. Of these, alkali metals such as lithium, sodium, potassium and the like are preferable, and lithium is more preferable.

The amount of these alkali metal or alkaline earth metal to be used is generally within the range of 0.7 to 20 times the amount necessary for reducing the carbon-carbon double bond of compound (I) to be reduced. When the amount of alkali metal or alkaline earth metal to be used is less than such range, reduction of the carbon-carbon double bond tends not to proceed sufficiently and to reduce the yield, and when it is greater than such range, side reactions (e.g., reduction of ketone and the like) tend to proceed further.

The reaction temperature is preferably within the range of -100°C to 50°C, more preferably within the range of -50°C to 20°C. While the reaction time varies depending on the reaction conditions, it is preferably within the range of 0.1 to 20 hr, more preferably within the range of 1 to 10 hr, from the industrial viewpoints.

The reduction reaction is carried out in the presence of ammonia and/or an amine. The kind of amine is not particularly limited and, for example, linear, branched or cyclic amines having 1 to 6 carbon atoms such as primary amines (e.g., methylamine, ethylamine, isopropylamine, butylamine and the like); secondary amines (e.g., dimethylamine, diethylamine, diisopropylamine, pyrrolidine, piperidine and the like); polyamines (e.g., ethylenediamine, diaminopropane, N,N'-dimethylethylenediamine and the like); and the like can be mentioned, with preference given to ammonia.

The amount of the ammonia and/or amine to be used is preferably within the range of 1- to 100-fold by mass, more preferably within the range of 3- to 50-fold by mass, relative to compound (I).

In addition, use of a proton donor is necessary for the reaction. While the kind of the proton donor is not particularly limited and, for example, inorganic acids such as hydrochloric acid, sulfuric acid, carbonic acid and the like, carboxylic acids such as formic acid, acetic acid, benzoic acid and the like, ammonium salts or amine salts thereof; water; alcohol and the like can be mentioned, with preference given to alcohol. The kind of the alcohol is not particularly limited and, for example, linear, branched or cyclic alcohols having 1 to 12 carbon atoms such as primary alcohols (e.g., methanol, ethanol, 1-propanol, 1-butanol, 1-octanol, 1-dodecanol and the like); secondary alcohols (e.g., 2-propanol, 2-butanol, 3-pentanol, cyclopentanol, cyclohexanol, 2-octanol and the like); tertiary alcohols (e.g., tert-butanol, tert-amylalcohol, 2-methylhexanol, 1-methylcyclohexanol and the like); polyhydric alcohols (e.g., ethylene glycol, 1,4-butanediol, 2,4-pentanediol, glycerol and the like); and the like can be mentioned. Of these, tertiary alcohol is preferable, and tert-butanol is more preferable.

The amount of the proton donor to be used is generally within the range of 1.5- to 3-fold by mol per one carbon-carbon double bond to be reduced.

The timing of the addition of the proton donor to the reaction system is not particularly limited and can be freely selected from, for example, a method including addition of a proton donor to the reaction system before reaction of compound (I) with an alkali metal or alkaline earth metal, a method including addition of a proton donor to the reaction system after reaction of compound (I) with an alkali metal or alkaline earth metal and the like, with preference given to the former method.

The reduction reaction may be carried out in the presence of a solvent. The usable solvents are not particularly limited as long as they do not adversely influence the reaction and, for example, ethers such as tetrahydrofuran, diethyl ether, diisopropyl ether, methyl tert-butyl ether, cyclopentyl methyl ether, dimethoxyethane, 1,4-dioxane and the like; saturated aliphatic hydrocarbons such as pentane, hexane, heptane, octane and the like; and the like can be mentioned. Of these, ethers such as tetrahydrofuran, diethyl ether, diisopropyl ether, methyl tert-butyl ether, dimethoxyethane, 1,4-dioxane and the like are preferable, and tetrahydrofuran is more preferable.

When a solvent is used, its amount of use is not particularly limited. However, it is preferably within the range of 1- to 100-fold by mass, more preferably within the range of 3- to 50-fold by mass, relative to compound (I).

By a reduction reaction, compound (I) is stereoselectively reduced such that the hydrogen atom at the 5-position of pregnane has an α, configuration. As used herein, by the stereoselectively is meant that compound (II) and compound (III) are produced in greater amounts than isomers wherein the hydrogen atom at the 5-position of pregnane has a β configuration.

The method of isolation and purification of compound (II) or compound (III) after the reduction reaction is not particularly limited, and methods generally used for the isolation and purification of organic compounds can be employed. For example, after extraction operation and the like, they are purified by recrystallization, column chromatography and the like as necessary.

The hydroxyl-protecting group represented by R¹ or R² in compound (I) may be the same as or different from the hydroxyl-protecting group represented by R¹¹ or R¹² in compound (II), or the hydroxyl-protecting group represented by R²¹ or R²² in compound (III). In other words, the hydroxyl-protecting group represented by R¹ or R² may optionally vary by carrying out a reduction reaction as long as it can be deprotected. For example, a benzoyl group may change to a 2,5-cyclohexadienecarbonyl group as a result of a reduction reaction. In addition, the hydroxyl-protecting group represented by R¹ or R² in compound (I) may be eliminated by carrying out a reduction reaction (Birch reduction reaction) during a step for producing a mixture of compound (II) from compound (I).

### 3. Deprotection method of hydroxyl-protecting group and reaction conditions (production method of compound (IV) from compound (III))

The reaction conditions used for elimination of the hydroxyl-protecting group represented by R²¹ or R²² in compound (III) is not particularly limited, and those generally used depending on the kind of the protecting groups can be selected for use.

For example, in the case of a tri-substituted silyl group for which a hydroxyl-protecting group is preferable, compound (III) is reacted with an acid or fluoride to allow deprotection. While this embodiment is explained in the following, the deprotection is not limited to the embodiment.

The kind of the acid is not particularly limited and, for example, inorganic acids such as hydrochloric acid, sulfuric acid, hydrofluoric acid, hydrobromic acid and the like; organic acids such as acetic acid, trifluoroacetic acid, p-toluenesulfonic acid, methanesulfonic acid and the like; and the like can be mentioned. As the fluorides, for example, tetrabutylammonium fluoride, potassium fluoride, sodium fluoride and the like can be mentioned.

The amount of the acid to be used is within the range of 0.01- to 10-fold by mol, more preferably within the range of 0.1- to 5-fold by mol, relative to compound (III).

The amount of the fluoride to be used is determined based on the number of the protecting groups contained in compound (III), which are to be eliminated. Preferably, it is within the range of 1- to 10-fold by mol, more preferably within the range of 1- to 5-fold by mol, relative to one protecting group.

The deprotection may be carried out in the presence of a solvent. Usable solvents are not particularly limited as long as they do not adversely influence the reaction and, for example, ethers such as tetrahydrofuran, diethyl ether, diisopropyl ether, methyl tert-butyl ether, cyclopentyl methyl ether, dimethoxyethane, 1,4-dioxane and the like; saturated aliphatic hydrocarbons such as pentane, hexane, heptane, octane and the like; and the like can be mentioned. Of these, ethers such as tetrahydrofuran, diethyl ether, diisopropyl ether, methyl tert-butyl ether, dimethoxyethane, 1,4-dioxane and the like are preferable, and tetrahydrofuran is more preferable.

When a solvent is to be used, its amount to be used is not particularly limited, but preferably within the range of 1- to 100-fold by mass, more preferably within the range of 3-to 50-fold by mass, relative to compound (III).

The reaction temperature is preferably within the range of -20°C to 120°C, more preferably within the range of 0°C to 80°C. The reaction time is not particularly limited, it is preferably within the range of 0.1 to 20 hr, more preferably within the range of 1 to 10 hr, from the industrial aspects.

The method of isolation and purification of compound (IV) obtained by a deprotection reaction is not particularly limited, and methods generally used for the isolation and purification of organic compounds can be employed. For example, after extraction operation and the like, it is purified recrystallization, column chromatography and the like as necessary.

### 4. Secure supply of raw material

The production method of compound (I) to be used as a raw material is not particularly limited. For example, (20S)-7α,21-dihydroxy-20-methylpregna-1,4-dien-3-one can be easily obtained by subjecting 3α,7α-dihydroxy-5β-cholanoic acid and/or a salt thereof to a conversion reaction using a microorganism (JP-B-2525049) to give 7α-hydroxy-3-oxo-pregna-1,4-diene-20_{α}-carbaldehyde, and reducing the 20-position of the compound with sodium borohydride (WO02/20552), and (20S)-7α,21-dihydroxy-20-methylpregn-4-en-3-one can be easily obtained by subjecting 3α,7α,-dihydroxy-5β-cholanoic acid to a conversion reaction using a microorganism to give 7α-hydroxy-3-oxo-pregn-4-ene-20α-carbaldehyde, and reducing the aldehyde group of the compound with sodium borohydride (WO03/23047). By protecting as necessary the hydroxyl groups at the 21-position and the 7-position of these compounds by a method known per se, compound (I) to be used in the present invention can be afforded.

### Examples

The present invention is explained in more detail in the following by referring to Examples, which are not to be construed as limitative.

### Production Example 1

### Production of (20S)-21-tert-butyldimethylsilyloxy-7α-hydroxy-20-methylpregna-1,4-dien-3-one

Under a nitrogen atmosphere, (20S)-7α,21-dihydroxy-20-methylpregna-1,4-dien-3-one (8.79 g, 25.5 mmol), imidazole (2.60 g, 38.3 mmol) and tetrahydrofuran (100 ml) were placed in a 200 ml flask, dissolved by stirring and ice-cooled. To this solution was added dropwise a solution of tert-butyldimethylchlorosilane (5.00 g, 33.2 mmol) dissolved in tetrahydrofuran (20 ml) while maintaining the inner temperature at 0°C to 10°C. After completion of the dropwise addition, the mixture was allowed to warm to room temperature and further stirred for 1 hr. The reaction solution was added to water (200 ml) and the mixture was extracted twice with ethyl acetate (100 ml). The aqueous layer was separated, and the organic layer was washed with saturated brine (100 ml), dried over anhydrous sodium sulfate and concentrated. The obtained crude product was purified by silica gel column chromatography to give (20S)-21-tert-butyldimethylsilyloxy-7α-hydroxy-20-methylpregna-1,4-dien-3-one (11.11 g, yield 95%) having the following property.
¹H-NMR spectrum (270MHz, CDCl₃, TMS, ppm) δ: 0.03 (s, 6H), 0.76(s, 3H), 0.89(s, 9H), 0.99(d, 3H, J=6.9Hz), 1.1-1. 8 (15H), 2.03(dt, 1H, J=3.0, 12.9Hz), 2.48(dd, 1H, J=3.0, 13.9Hz), 2.75(dt, 1H, J=2.0, 13.9Hz), 3.28(dd, 1H, J=6.9, 9.9Hz), 3.56(dd, 1H, J=3.0, 9.9Hz), 4.05(bs, 1H), 6.14(dd, 1H, J=0.9, 2.0Hz), 6.24(dd, 1H, J=2.0, 9.9Hz), 7.08(d, 1H, J=9.9Hz).

### Production Example 2

### Production of (20S)-21-tert-butyldimethylsilyloxy-7α-hydroxy-20-methylpregn-4-en-3-one

Under a nitrogen atmosphere, (20S)-7α,21-dihydroxy-20-methylpregn-4-en-3-one (7.70 g, 22.2 mmol), imidazole (2.27 g, 33.3 mmol) and dichloromethane (70 ml) were placed in a 200 ml flask, dissolved by stirring and ice-cooled. To this solution was added dropwise tert-butyldimethylchlorosilane (4.02 g, 26.7 mmol) while maintaining the inner temperature at 0°C to 10°C. After completion of the dropwise addition, the mixture was allowed to warm to room temperature and further stirred for 1 hr. The reaction solution was poured into water (100 ml) and the mixture was extracted twice with ethyl acetate (100 ml). The aqueous layer was separated, and the organic layer was washed with saturated brine (100 ml), dried over anhydrous sodium sulfate and concentrated. The obtained crude product was purified by silica gel column chromatography to give (20S)-21-tert-butyldimethylsilyloxy-7αhydroxy-20-methylpregn-4-en-3-one (6.73 g, yield 66%) having the following property. ¹H-NMR spectrum (270MHz, CDCl₃, TMS, ppm) δ: 0.03(s, 6H), 0.73(s, 3H), 0.89(s, 9H), 0.99(d, 3H, J=6.9Hz), 1.19 (s, 3H), 1.13-2.07 (15H), 2.37-2.44(3H), 2.63(d, 1H, J=14.8Hz), 3.27(dd, 1H, J=6.9, 9.9Hz), 3.57(dd, 1H, J=3.0, 9.9Hz), 4.00(bs, 1H), 5.80 (s, 1H)

### Example 1

### Production of (20S)-21-tert-butyldimethylsilyloxy-7α-hydroxy-20-methyl-5α-pregn-3-one

Under a nitrogen atmosphere, tetrahydrofuran (85 ml), (20S)-21-tert-butyldimethylsilyloxy-7α-hydroxy-20-methylpregna-1,4-dien-3-one (5.00 g, 10.9 mmol) and tert-butanol (3.55 g, 47.9 mmol) were placed in a 300 ml three-necked flask. The mixture was cooled to below -50°C and liquid ammonia (85 ml) was added. Then, metal lithium (0.38 g, 55.0 mmol) was slowly added while maintaining the inner temperature at -50°C to -40°C. After completion of the addition, the mixture was further stirred at -40°C for 3 hr. Ammonium acetate (4.23 g, 55.0 mmol) was added to the reaction solution, and the mixture was stirred for 12 hr to remove ammonia while allowing the reaction mixture to warm to room temperature. To the obtained tetrahydrofuran solution was added 15% by mass of an aqueous sulfuric acid solution to adjust the pH of the aqueous layer to 4 to 6, and the organic layer was separated from the aqueous layer. The organic layer was washed with saturated brine, dried over anhydrous magnesium sulfate and concentrated. The obtained crude product was purified by silica gel column chromatography to give (20S)-21-tert-butyldimethylsilyloxy-7α,-hydroxy-20-methyl-5α-pregn-3-one (4.79 g, yield 95%) having the following property.
¹H-NMR spectrum (270MHz, CDCl₃, TMS, ppm) δ: 0.03 (s, 6H), 0.71(s, 3H), 0.88 (s, 9H), 0.98(d, 3H, J=6.9Hz), 1.00(s, 3H), 1.1-2.4(22H), 3.28(dd, 1H, J=6.9, 10.9Hz), 3.56(dd, 1H, J=3.0, 10.9Hz), 3.87(bs, 1H).

### Example 2

### Production of (20S)-21-tert-butyldimethylsilyloxy-7α-hydroxy-20-methyl-5α-pregn-3-one

Under a nitrogen atmosphere, tetrahydrofuran (100 ml), (20S)-21-tert-butyldimethylsilyloxy-7α-hydroxy-20-methylpregn-4-en-3-one (5.00 g, 10.9 mmol) and tert-butanol (1.78 g, 24.0 mmol) were placed in a 300 ml three-necked flask. The mixture was cooled to below -50°C and liquid ammonia (100 ml) was added. Then, metal lithium (0.17 g, 24.0 mmol) was slowly added while maintaining the inner temperature at -50°C to - 40°C. After completion of the addition, the mixture was further stirred at -40°C for 3 hr. Ammonium sulfate (1.59 g, 12.0 mmol) was added to the reaction solution, and the mixture was stirred for 12 hr to remove ammonia while allowing the reaction mixture to warm to room temperature. To the obtained tetrahydrofuran solution was added 15% by mass of an aqueous sulfuric acid solution to adjust the pH of the aqueous layer to 4 to 6, and the organic layer was separated from the aqueous layer. The organic layer was washed with saturated brine, dried over anhydrous magnesium sulfate and concentrated. The obtained crude product was purified by silica gel column chromatography to give (20S)-21-tert-butyldimethylsilyloxy-7α-hydroxy-20-methyl-5α-pregn-3-one (4.82 g, yield 96%).

### Example 3

### Production of (20S)-7α,21-dihydroxy-20-methyl-5α-pregn-3-one

Under a nitrogen atmosphere, (20S)-21-tert-butyldimethylsilyloxy-7α-hydroxy-20-methyl-5α-pregn-3-one (4.63 g, 10.0 mmol), tetrahydrofuran (30 ml) and 6N hydrochloric acid (2 ml) were placed in a 100 ml three-necked flask. The mixture was stirred at 40°C for 2 hr. After confirmation of disappearance of the raw material by TLC, 10% by mass of an aqueous sodium hydroxide solution (10 ml) was added. Toluene (30 ml) was added thereto, tetrahydrofuran was removed by heating under atmospheric pressure, and the mixture was cooled to below 30°C and filtered. The filtrate was washed twice with water (10 ml), washed twice with toluene (10 ml) and dried in vacuo to give (205)-7α,21-dihydroxy-20-methyl-5α-pregn-3-one (3.31 g, yield 95%) having the following property.
¹H-NMR spectrum (270MHz, CDCl₃, TMS, ppm) δ: 0.71(s, 3H), 1.01 (s, 3H), 1.04 (d, 3H, J=6.9Hz), 1.0-2.5 (22H),3. 34 (dd, 1H, J=6.9, 10.9Hz), 3.61(dd, 1H, J=3.0, 10.9Hz), 3.84-3.85(brs, 1H) .

### Industrial Applicability

Compound (II) and compound (IV) ((20S)-7α,21-dihydroxy-20-methyl-5α-pregn-3-one) produced by the present invention can be easily converted to squalamine by the method described in WO01/7925 Therefore, the method of the present invention can be advantageously used for the production of synthetic intermediates for squalamine.

This application is based on patent application No. 108419/2004 filed in Japan on March 31, 2004, the contents of which are hereby incorporated by reference.

## Claims

1. A method for producing a 5α-pregnane derivative represented by the formula (II): 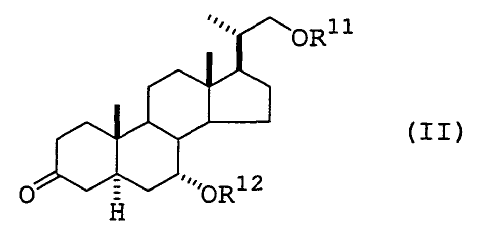 wherein R¹¹ and R¹² are each independently a hydrogen atom or a hydroxyl-protecting group, which comprises reacting a pregnane derivative represented by the formula (I): 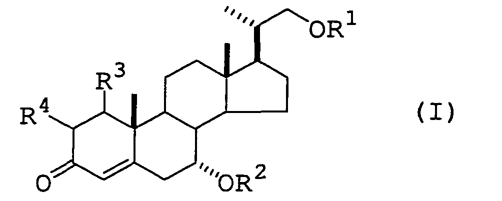 wherein R¹ is a hydroxyl-protecting group, R² is a hydrogen atom or a hydroxyl-protecting group, and R³ and R⁴ are each a hydrogen atom or in combination form a bond, with a metal selected from alkali metals and alkaline earth metals in the presence of a proton donor and an amine and/or ammonia.

2. The method of claim 1, wherein R² and R¹² are hydrogen atoms.

3. The method of claim 1 or 2, wherein R³ and R⁴ in combination form a bond.

4. The method of claim 3, wherein R¹ and R¹¹ are tri-substituted silyl groups having three, same or different, substituents selected from the group consisting of an alkyl group optionally having substituent(s), an aryl group optionally having substituent(s), an alkoxyl group optionally having substituent(s) and an aryloxy group optionally having substituent(s).

5. The method of claim 4, wherein R¹ and R¹¹ are tert-butyldimethylsilyl groups.

6. The method of any one of claims 1 to 5, wherein the metal is an alkali metal.

7. The method of claim 6, wherein the alkali metal is lithium.

8. A method for producing (20S)-7α,21-dihydroxy-20-methyl-5α-pregn-3-one represented by the formula (IV): 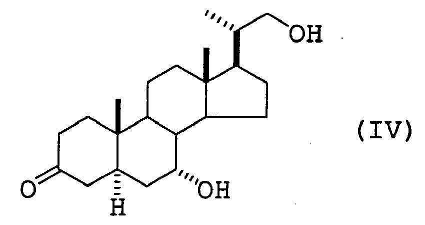 , which comprises the steps of
(a) reacting a pregnane derivative represented by the formula (I) : 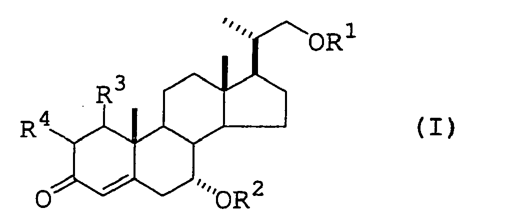 wherein R¹ is a hydroxyl-protecting group, R² is a hydrogen atom or a hydroxyl-protecting group, and R³ and R⁴ are each a hydrogen atom or in combination form a bond, with a metal selected from alkali metals and alkaline earth metals in the presence of a proton donor and an amine and/or ammonia to give a 5α-pregnane derivative represented by the formula (III): 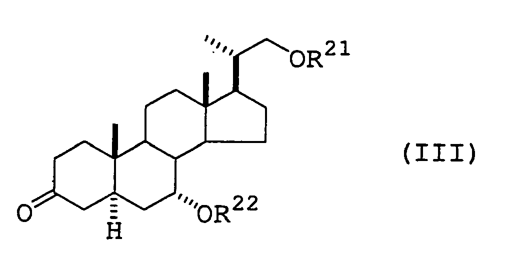 wherein R²¹ is a hydroxyl-protecting group and R²² is a hydrogen atom or a hydroxyl-protecting group; and
(b) eliminating the hydroxyl-protecting group of the 5α-pregnane derivative represented by the formula (III) obtained by the aforementioned step.

9. The method of claim 8, wherein R² and R²² are hydrogen atoms.

10. The method of claim 8 or 9, wherein R³ and R⁴ in combination form a bond.

11. The method of claim 10, wherein R¹ and R²¹ are tri-substituted silyl groups having three, same or different, substituents selected from the group consisting of an alkyl group optionally having substituent(s), an aryl group optionally having substituent(s), an alkoxyl group optionally having substituent(s) and an aryloxy group optionally having substituent (s).

12. The method of claim 11, wherein R¹ andR²¹ are tert-butyldimethylsilyl groups.
